(19) Europäisches Patentamt
European Patent Office
Office européen des brevets

(11) Veröffentlichungsnummer: **0 191 940 B1**

(12) **EUROPÄISCHE PATENTSCHRIFT**

(45) Veröffentlichungstag der Patentschrift: **13.03.91**

(51) Int. Cl.5: **C07D 235/32**

(21) Anmeldenummer: **85116204.0**

(22) Anmeldetag: **18.12.85**

# (54) Verfahren zur Herstellung von Benzimidazol-thiolen.

(30) Priorität: **19.12.84 HU 472584**

(43) Veröffentlichungstag der Anmeldung:
**27.08.86 Patentblatt 86/35**

(45) Bekanntmachung des Hinweises auf die Patenterteilung:
**13.03.91 Patentblatt 91/11**

(84) Benannte Vertragsstaaten:
**AT CH DE FR GB IT LI**

(56) Entgegenhaltungen:
**EP-A- 0 031 473**
**DE-A- 2 820 375**

(73) Patentinhaber: **CHINOIN Gyogyszer és Vegyészeti Termékek Gyára RT.**
**To utca 1-5**
**H-1045 Budapest IV(HU)**

(72) Erfinder: **Bakonyi, Mária**
**Munkacsy Mihaly u. 58/a**
**H-1046 Budapest(HU)**
Erfinder: **Lugosi, György**
**9. J zsef u.**
**H-2132 Göd-felsö(HU)**
Erfinder: **Kállai, Tamás**
**16. Gépmadár u.**
**H-1106 Budapest(HU)**
Erfinder: **Hima, Mária**
**35. Tito u.**
**H-1042 Budapest(HU)**
Erfinder: **Montay, Tibor**
**123. Baross u.**
**H-1089 Budapest(HU)**
Erfinder: **Sziládi, Mária**
**12. Attila u.**
**H-1152 Budapest(HU)**

(74) Vertreter: **Bartsch, Elisabeth, Dr.**
**Patentanwälte Lotterhos & Partner Lichtensteinstrasse 3**
**W-6000 Frankfurt am Main 1(DE)**

**EP 0 191 940 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues Verfahren zur Herstellung von Benzimidazol-thiol-derivaten der allgemeinen Formel:

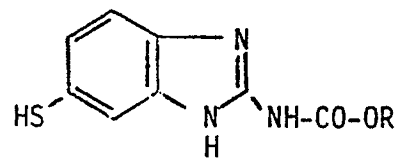

(I) ,

worin

R $C_{1-5}$-alkyl bedeutet,

durch Reduktion von Verbindungen der allgemeinen Formel:

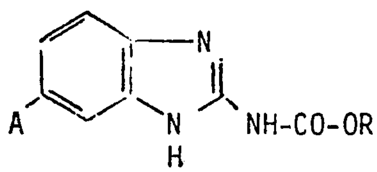

(II) ,

worin

A $-SO_2X$, -SOH, $-SOA^1$ oder $-SA^1$,

X Chlor oder Wasserstoff und

$A^1$ eine Gruppe der allgemeinen Formel:

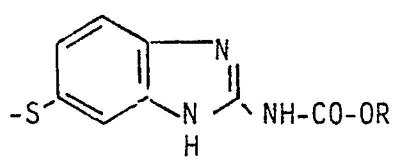

($A^1$)

bedeutet und

R die oben angegebene Bedeutung hat,

und/oder deren Salzen mittels eines Metalls in Gegenwart von Wasser und einer Mineralsäure. Das Reduktionsverfahren gemäß Erfindung wird in einer Mischung von Wasser, Mineralsäure und aliphatischer Carbonsäure mit 1 - 3 C-Atomen mit Hilfe von Aluminium, das bis zu 5 Gew.% - auf das Aluminium bezogen - durch Blei, Magnesium, Zink, Quecksilber und/oder Eisen, gewünschtenfalls in Form eines Salzes aktiviert worden ist, bei einer Temperatur von 0 - 100 °C durchgeführt. Das so erhaltene Produkt kann danach - falls gewünscht - aus einem sauren Medium, vorzugsweise bei einem pH von 2 - 3, in kristalliner Form isoliert werden.

Die Definition der Substituenten R, A, X und $A^1$ ist in der gesamten Beschreibung die gleiche.

Die Verbindungen der allgemeinen Formel I sind bekannt. Sie sind Zwischenprodukte im Verfahren zur Herstellung der biologisch aktiven 2-(Alkoxy-carbonyl-amino)-benzimidazol-derivate. Bislang sind nur zwei Verfahren zur Herstellung und Isolierung der Produkte bekannt geworden.

Nach dem Verfahren der HU-PS 177 418 wird bis-[(2-Methoxycarbonyl)-amino-benzimidazol-5(6)-yl]-disulfid mit metallischem Natrium in flüssigem Ammoniak reduziert und dann das Thiol aus dem Natriumsalz der Thiolverbindung, das nach dem Verdampfen des Ammoniaks als Rückstand erhalten wird, freigesetzt. Der Nachteil dieses Verfahrens besteht darin, daß es industriell schwierig durchführbar ist. Es erfordert nicht nur eine kostspielige Ausrüstung, darüber hinaus müssen spezielle Sicherheitsmaßnahmen eingehalten werden, da die Handhabung von metallischem Natrium und von flüssigem Ammoniak mit Gefahren verbunden ist. Das nach dem Ausführungsbeispiel erhaltene Produkt hat einen Schmelzpunkt von 260 - 262 °C.

Nach dem Verfahren der HU-PS 182 782 wird das Produkt aus dem Hydrochloridsalz des Sulfonsäurechlorids, das unter die allgemeine Formel II fällt, durch Reduktion mit Metallen in Gegenwart von Wasser und Mineralsäure erhalten. Als Reduktionsmittel werden Zinn, Zink und Eisen verwendet. In allen Fällen

führt die Reduktion zu den entsprechenden Metallsalzen der Thiole der allgemeinen Formel I, die aus der gegebenen Reaktionsmischung ausfallen und so isoliert werden konnen. Nach dem einzigen Beispiel, das die Herstellung des isolierten Thiols betrifft, wurde das Zinnsalz des Thiols, das als Reduktionsprodukt erhalten wurde, durch heiße Natriumcarbonatlösung zersetzt, wobei unlösliches Zinnhydroxyd ausfiel, das abfiltriert wurde. Aus dem so erhaltenen Filtrat wurde dann das Thiol isoliert. Dieses Verfahren ist langwierig und mit großen Verlusten verbunden, da das Thiol innerhalb kurzer Zeit zu dem entsprechenden Disulfid oxydiert. Ein weiteres Problem besteht darin, daß es sehr schwierig ist, die Zinnverunreinigung aus dem Produkt zu entfernen. Dies kann nur in mehreren Stufen gelöst werden, was zu Verlusten führt. Zur Umgehung der mit der Isolierung der Thiole verbundenen Schwierigkeiten wurde nach dem Verfahren dieser Patentschrift die erhaltenen Metallsalze weiter umgesetzt. Die Metallverunreinigungen konnten dann in einer weiteren Reaktionsstufe entfernt werden.

Von den in dem Verfahren dieser Patentschrift verwendeten Metallen liefert Zinn die höchsten Ausbeuten. Da jedoch der Preis von Zinn hoch ist, ist ein solches Verfahren unwirtschaftlich. Zink hat dagegegen eine starke Neigung zur Komplexbildung, was mit verschiedenen Problemen verbunden ist, während bei Verwendung von Eisen die Ausbeute nicht zufriedenstellend ist.

In Anbetracht dieser Schwierigkeiten ist es überraschend, daß in dieser Patentschrift die Verwendung von Aluminium nicht erwähnt worden ist.

Bei den Untersuchungen zur Verbesserung der bekannten Verfahren zur Herstellung der Thiole der allgemeinen Formel I wurde nach einem einfachen, wirtschaftlich durchführbaren Verfahren gesucht, das die Produktion der gewünschten Thiole in einfacher Weise in industriellem Maßstab ermöglicht und ein reines Produkt liefert.

Bei diesen Untersuchungen wurde überraschender Weise gefunden, daß sich Aluminium für diese Reduktion außerordentlich gut eignet und daß bei Einsatz von Aluminium ein reines Endprodukt - selbst beim Arbeiten in industriellem Maßstab - erzielt werden kann. Um bessere Ausbeuten zu erzielen, ist es erforderlich, entsprechend aktiviertes Aluminium, z.B. Aluminiumfolien mit großer Oberfläche, zu verwenden. Cruder, Aluminiumsorten des Handels, z.B. Aluminium-Grieß, -Stückchen oder irgendeine Art von Flitter, können auch verwendet werden, aber nur dann, wenn sie in geeigneter Weise aktiviert sind, da sonst keine zufriedenstellende Ausbeute erzielt werden kann.

Gemäß Erfindung können zur Aktivierung - wie gefunden wurde -
vorzugsweise Zinn, Blei, Magnesium, Zink, Quecksilber oder Eisen,
in Form der Salze, verwendet werden. Andere Metalle sind - wie die Untersuchungen ergeben haben - für diesen Zweck nicht geeignet.

Diese Metalle oder Metallmischungen können auch in Form ihrer Salze verwendet werden, da sie in dem gegebenen Reduktionsmedium in Metalle übergeführt werden. Die Menge des aktivierend wirkenden Metalls beträgt praktisch bis zu 5 Gew.% bezogen auf Aluminium.

Bei Verwendung von Zinn als Aktivator in dem Verfahren gemäß Erfindung ist es sehr zweckmäßig, das nicht umgesetzte Aluminium und Zinn wieder in das Verfahren zurückzuführen, nach dem die verbrauchte Menge Aluminium ergänzt worden ist. Auf diese Weise ist es möglich, bei einem kontinuierlichen Verfahren das in das Verfahren eingeführte Zinn theoretisch ohne Verluste, praktisch nur mit einigen Recyclingsverlusten zu verwenden.

Das Verfahren der Erfindung basiert weiterhin auf der Erkenntnis, daß auch das Reaktionsmedium einen bedeutenden Einfluß auf die Steigerung der Ausbeuten hat. Es wurde gefunden, daß es nicht ausreicht, nur zu gewährleisten, daß das Reaktionsmedium sauer ist, es ist vielmehr einer Mischung von einer wäßrigen Mineralsäure, vorzugsweise Bromwasserstoffsäure, und einer organischen Säure mit 1 - 3 C-Atomen, vorzugsweise Ameisensäure, der Vorzug zu geben. Werden Sulfochloride als Ausgangsmaterialien verwendet, hindert die organische Säure die Ausfällung der Disulfide, die als Zwischenprodukte gebildet werden und in diesem Medium schlecht löslich sind.

In dem Verfahren gemäß Erfindung wird als gut isolierbares Endprodukt das reine Thiol erhalten. Im Gegensatz dazu wird bei dem Verfahren der HU-PS 182 782, bei dem Zinn oder Zink verwendet wird, das Metallsalz des Thiols erhalten, dessen Reinigung schwierig ist.

Da der Schmelzpunkt des gemäß Erfindung erhaltenen Produkts der allgemeinen Formel I, in der R Methyl ist, 435 °C beträgt, und damit sehr viel höher als der Schmelzpunkt des Produkts ist, der bei dem Verfahren der HU-PS 177 418 erhalten wird, und da in der HU-PS 182 782 keine Schmelzpunktangaben enthalten sind - die Endprodukte sind durch die Daten der Elementaranalyse charakterisiert (die keine Anhaltspunkte für die Anwesenheit des Disulfid-derivates der allgemeinen Formel II liefern, was auf die Gleichartigkeit in der elementaren Zusammensetzung zurückzuführen ist) - wurde es für notwendig gehalten, das Produkt gründlich zu analysieren.

Auber der Bestimmung der elementaren Zusammensetzung wurde das Molekulargewicht mittels der

Massenspektroskopie bestimmt. Außerdem wurde eine Methode zur Bestimmung des Reinheitsgrades des Produkts mittels Dünnschichtchromatographie ausgearbeitet, wobei in besonderem Maße die Anwesenheit des Disulfids der allgemeinen Formel II als mögliche Verunreinigung berücksichtigt wurde. Es wurde gefunden, daß das Molgewicht dieses Produkts 223 beträgt, was der Verbindung der allgemeinen Formel I, in der R Methyl ist, entspricht. Nach dem Ergebnis der Dünnschichtchromatographie bzw. des $R_f$-Wertes enthält dieses Produkt als Verunreinigung 1 - 2 Gew.% des Disulfids der allgemeinen Formel II.

Aus dem gemäß Erfindung erhaltenen Endprodukt (nach Isolierung oder auch ohne Isolierung) wurde [5-Propylthio)-1H-Benzimidazol-2-yl]-carbaminsäure-methylester durch Propylierung in hoher Reinheit und in einer Ausbeute von 90% erhalten.

Die Erfindung betrifft auch 2-[(Methoxycarbonyl)-amino]-1H-benzimidazol-5(6)-thiol mit einem Schmelzpunkt von nahezu 435 °C sowie dessen Salze, erhältlich durch Reduktion einer Verbindung der allgemeinen Formel II oder deren Salzen, worin A, X und $A^1$ die in Formel I angegebene Bedeutung haben und R = $CH_3$ ist, mit Hilfe von Aluminium, das bis zu 5 Gew.% - auf das Aluminium bezogen - durch Zinn, Blei, Magnesium, Zink, Quecksilber und/oder Eisen, gewünschtenfalls in Form eines Salzes, aktiviert ist, in einer Mischung von Wasser, Mineralsäure und einer aliphatischen $C_{1-3}$-Carbonsäure und danach - falls gewünscht - Isolierung des erhaltenen Produktes aus einem sauren Medium, vorzugsweise im pH-Bereich von 2 - 3, in kristalliner Form.

Das Verfahren der Erfindung wird durch die folgenden Beispiele näher erläutert. Aus den Beispielen ist keine Beschränkung herzuleiten.

## Beispiel 1

32,62 g 2-[(Methoxycarbonyl)-amino]-1H-benzimidazol-5-sulfonylchlorid-Hydrochlorid-salz gibt man unter Rühren zu einer Mischung von 80 ml Ameisensäure, 120 ml Wasser und 40 ml 50 %iger Bromwasserstoffsäure und fügt danach der so erhaltenen Reaktionsmischung 10 g in Stücke gerissene Aluminiumfolie zu. Die Aluminiumzugabe ist von einer leichten Wasserstoffentwicklung begleitet.

Die Reaktionsmischung wird 1,5 Stunden bei einer Temperatur von 35 - 40 °C, dann 30 Minuten bei einer Temperatur von 60 - 65 °C gerührt und danach auf eine Temperatur von +5 °C gekühlt. Danach filtriert man ungelöstes Aluminium (3 g) ab und stellt den pH-Wert des Filtrats durch Zusatz von 80 ml 40 %iger Natriumhydroxydlösung auf 2 - 3 ein. Die ausgefallenen weißen Kristalle werden abfiltriert, mit Wasser gewaschen, mit Methanol bedeckt und getrocknet.

Es werden 19,6 g 2-[(Methoxycarbonyl)-amino]-1H-benzimidazol-5(6)-thiol erhalten.

Ausbeute: 87 %

Schmelzpunkt: 435 °C.

Aschengehalt: 0,2 - 0,5 %. Nach dem Ergebnis der Dünnschichtchromatographie ist das Endprodukt nur durch eine einzige Substanz verunreinigt. Bei dieser Verunreinigung handelt es sich nach dem $R_f$-Wert um 1 - 2 % bis-[2-Methoxycarbonyl)-amino)-benzimidazol-5(6)-yl]-disulfid.

Analyse:

| | | | | | | | | |
|---|---|---|---|---|---|---|---|---|
| berechnet: | C % | 48,20 | H % | 3,90 | N % | 18,6 | S % | 14,30 |
| gefunden: | C % | 48,42 | H % | 4,06 | N % | 18,82 | S % | 14,36 . |

## Beispiel 2

32,62 g 2-[(Methoxycarbonyl)-amino]-1H-benzimidazol-5-sulfonylchlorid-Hydrochlorid-salz suspendiert man in 120 ml Wasser, gibt dann 15 g Aluminiumstücke von 2 bis 3 cm (im Handel erhältlicher Abfall) und 0,5 g Zinnpulver zu und rührt die Mischung 20 Minuten bei einer Temperatur von 10 - 15 °C.

In einem getrennten Gefäß vermischt man 80 ml 99 %iger Ameisensäure mit 40 ml 50 %iger wäßriger Bromwasserstoffsäure und tropft die erhaltene Lösung innerhalb von einer halben Stunde bei einer Temperatur von 10 - 15 °C unter Kühlung mittels einer Salzlösung zu der Reaktionsmischung. Danach wird die Reaktionsmischung zunächst 1 1/2 Stunden bei einer Temperatur von 35 - 40 °C und dann eine halbe Stunde bei einer Temperatur von 60 - 65 °C gerührt. Die ungelösten Metallteilchen (8 g) werden aus der kalten Lösung abfiltriert.

Den pH-Wert des Filtrats stellt man durch Zusatz von 80 ml 40 %iger wäßriger Natriumhydroxydlösung unter Kühlung auf 2 - 3 ein, filtriert den ausgefallenen weißen Niederschlag ab, wäscht ihn mit Wasser und Methanol und trocknet ihn.
Man erhält 20 g 2-[(Methoxycarbonyl)-amino]-1H-benzimidazol-5(6)-thiol als Endprodukt.
Ausbeute: 90 %. Das erhaltene Produkt stimmt in den Eigenschaften mit denen des Produkts des Beispiels 1 überein.

Beispiel 3

32,62 g des Hydrochloridsalzes von 2-[(Methoxycarbonyl)-amino]-1H-benzimidazol-5-sulfonylchlorid suspendiert man in 120 ml Wasser, gibt den in Beispiel 2 nach Beendigung der Reaktion abfiltrierten Rückstand von 8 g ungelöstem Metall (Zinn und Aluminium) sowie 7 g im Handel erhältliche Aluminiumflitter zu und rührt die Mischung 20 Minuten bei einer Temperatur von 10 - 15 °C.
Das weitere Verfahren erfolgt in der in Beispiel 2 angegebenen Weise. Man erhält so 20,14 g 2-[(Methoxycarbonyl )-amino]-1H-benzimidazol-5(6)-thiol.
Das erhaltene Produkt stimmt in den Eigenschaften mit denen des Produkts des Beispiels 1 überein.

Beispiel 4

Es wird wie in Beispiel 2 verfahren, jedoch mit der Ausnahme, daß anstelle von Ameisensäure in der Reaktion Essigsäure derselben Menge verwendet wird. Die Isolierung des gewünschten Produkts kann in der folgenden Weise erfolgen:
a) die ungelösten Metalle filtriert man nach der Reduktion aus der Reaktionsmischung ab, verdünnt das erhaltene Filtrat mit 1500 ml Wasser, filtriert die ausgefallenen weißen Kristalle ab, wäscht sie mit Wasser und Methanol und trocknet sie.
Man erhalt so 16,7 g 2-[(Methoxycarbonyl )-amino]-1H-benzimidazol-5(6)-thiol.
b) Nach Beendigung der Reduktion filtriert man die ungelösten Metalle ab, stellt danach den pH-Wert der Reaktionsmischung unter Kühlen durch tropfenweise Zugabe von etwa 70 ml 40 %iger wäßriger Natriumhydroxydlösung bei Raumtemperatur auf 5 ein und isoliert die ausgefallenen Kristalle.
Man erhält so 19 g 2-[(Methoxycarbonyl)-amino]-1H-benzimidazol-5(6)-thiol. Das so erhaltene Produkt stimmt in den Eigenschaften mit denen des Produkts des Beispiels 2 überein.

Beispiel 5

Zu der Lösung von 80 ml 99 %iger Ameisensäure, 120 ml Wasser und 40 ml 50 %iger wäßriger Bromwasserstoffsäure gibt man 6 g Aluminiumflitter und rührt die Reaktionsmischung 15 Minuten bei einer Temperatur von 10 - 15 °C. Dann gibt man 0,3 g Zinnpulver und 22,2 g bis-[2-(Methoxycarbonyl-amino)-benzimidazol-5(6)-yl]-disulfid zu und rührt die Lösung 1,5 Stunden bei einer Temperatur von 30 - 40 °C. (Bei Beginn der Reaktion kann eine leichte Gas- und Wärmeentwicklung beobachtet werden, die später aufhört und gelindes Erwärmen notwendig macht, um die geeignete Temperatur aufrecht zu erhalten.) Dann kühlt man die Reaktionsmischung auf eine Temperatur von 5 °C ab, filtriert die ungelösten Metalle ab und stellt den pH-Wert des Filtrats durch Zusatz von 80 ml 40 %iger wäBriger Natriumhydroxydlösung auf 2 - 3 ein. Die ausgefallenen weißen Kristalle filtriert man ab, wäscht sie mit Wasser und Methanol und trocknet sie.
Man erhält so 20,05 g 2-[(Methoxycarbonyl)-amino]-1H-benzimidazol-5(6)-thiol.
Das erhaltene Produkt stimmt in seinen Eigenschaften mit denen des Produkts des Beispiels 1 überein.

Beispiele 6 bis 12

Es wurde wie in Beispiel 2 verfahren, jedoch mit der Ausnahme, daß die in der folgenden Tabelle angegebenen Mineralsäuren, aktivierend wirkenden Additive und Reduktionstemperaturen angewendet wurden.

| Nr. des Beispiels | Mineralsäure | Reaktions-temperatur °C | aktivierend wirkende Additive | Aus-beute g |
|---|---|---|---|---|
| 6 | 40 ml 50 % wäßrige HBr | 35 - 65 | 1 g $SnCl_2.2H_2O$ | 19,8 |
| 7 | 60 ml 50 % wäßrige HBr | 35 - 40 | 0,5 g Sn-Pulver | 19,9 |
| 8 | 33 ml cc. HCl | 35 - 65 | 0,5 g Sn-Pulver | 16,7 |
| 9 | 40 ml 50 % wäßrige HBr | 35 - 65 | 1,2 g $Pb(CH_3COO)_2$ | 19,0 |
| 10 | 40 ml 50 % wäßrige HBr | 20 - 60 | 4 g $HgCl_2$ | 12,3 |
| 11 | 40 ml 50 % wäßrige HBr | 55 - 70 | 2 g Fe-Pulver | 13,0 |
| 12 | 40 ml 50 % wäßrige HBr | 55 - 70 | 3 g $FeSO_4.5H_2O$ | 13,5 |

Bemerkung: Bei Verwendung von Kupfer- und Nickelsalzen als Additive wurden keine zufriedenstellenden Ergebnisse erzielt.

Beispiel 13 Transformation des Produkts (nicht Gegenstand der Erfindung)

22,3 g 2-[(Methoxycarbonyl)-amino]-1H-benzimidazol-5(6)-thiol suspendiert man in 200 ml Wasser und kühlt die Suspension auf 10 °C. Dann gibt man 80 ml 40 %ige wäßrige Natriumhydroxydlösung unter Kühlung zu, worauf das Thiol der Formel I in Lösung geht. Die Reaktionsmischung wird durch Behandlung mit 1 g Aktivkohle bei einer Temperatur von 10 - 15 °C geklärt und filtriert. Dann gibt man 100 ml Methanol und 14 ml n-Propylbromid zu dem Filtrat, erhitzt es auf 40 - 35 °C und rührt eine Stunde. Nach einigen Minuten beginnt [5-(Propylthio)-1H-benzimidazol-2-yl)-carbaminsäuremethylester auszufallen. Nach Beendigung der Reaktion filtriert man die weiße kristalline Substanz ab, wäscht sie mit 300 ml Wasser, schlämmt sie mit 100 ml 5 %iger wäßriger Ameisensäure auf, filtriert sie ab, wäscht sie mit Wasser neutral, bedeckt sie mit Methanol, filtriert sie ab und trocknet sie bei einer Temperatur von 50 °C.
Man erhält so 23,8 g des gewünschten Endprodukts, was 90 % der berechneten Ausbeute entspricht.
Das erhaltene Produkt entspricht in seinen Eigenschaften den internationalen Standard - Anforderungen an die medizinischen Wirkstoffe.

Vergleichsbeispiele

Beispiel 14

Es wird wie in Beispiel 2 verfahren, jedoch mit der Ausnahme, daß Ameisensäure in der Reaktion nicht verwendet wird und daß die Menge an 50 %iger wäßriger Bromwasserstoffsäure von 40 ml auf 80 ml erhöht wird.
Das hat zur Folge, daß während der Reaktion unlösliches HBr-salz des Disulfids der allgemeinen Formel II ausfällt, das am Ende der Reaktion zusammen mit den ungelösten Metallen abfiltriert wird. Das Produkt wird aus dem Filtrat in der in Beispiel 2 angegebenen Weise isoliert. Das Gewicht des erhaltenen Produkts beträgt 6,7 g, was 30 % der berechneten Ausbeute entspricht.

Beispiel 15

Es wird wie in Beispiel 2 verfahren, jedoch mit der Ausnahme, daß Zinn in der Reduktion nicht benutzt wird.
Es werden 20 g eines Produktes erhalten, das eine Mischung des Thiols der allgemeinen Formel I und des Disulfids, das unter die allgemeine Formel II fällt, darstellt.
Das so erhaltene Produkt gibt man unter Rühren zu 200 ml 10 %iger wäßriger Natriumhydroxydlösung, wobei das Thiol der allgemeinen Formel I in Lösung geht, während das Disulfid der allgemeinen Formel II ungelöst bleibt, so daß die beiden Substanzen durch Filtration getrennt werden können. Durch Einstellung des pH-Werts der Lösung kann das Thiol der allgemeinen Formel I gefällt werden.
Die Ausbeute beträgt 40 - 70 %, was von der Aktivität des verwendeten Aluminiums abhängt.

## Ansprüche

1. Verfahren zur Herstellung von Benzimidazol-thiol-derivaten der allgemeinen Formel

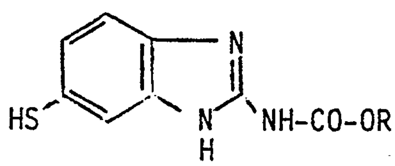

(I) ,

worin
R $C_{1-5}$-Alkyl bedeutet,
durch Reduktion einer Verbindung der allgemeinen Formel II oder deren Salze

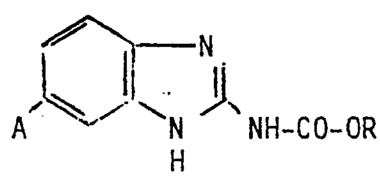

(II) ,

worin
A $-SO_2X$, -SOH, $-SOA^1$ oder $-SA^1$,
X Chlor oder Wasserstoff und
$A^1$ eine Gruppe der allgemeinen Formel:

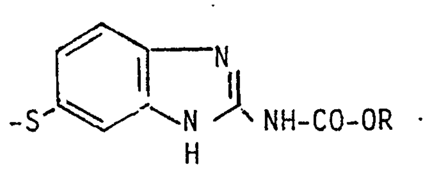

($A^1$)

bedeutet und
R die oben angegebene Bedeutung hat,
mittels eines Metalls in Gegenwart von Wasser und einer Mineralsäure im Temperaturbereich von 0 bis 100° C, dadurch gekennzeichnet, daß man
eine Verbindung der allgemeinen Formel II,
worin A, X, $A^1$ und R die oben angegebene Bedeutung haben, mit Hilfe von Aluminium, das bis zu 5 Gew.% - auf das Aluminium bezogen - durch Zinn, Blei, Magnesium, Zink, Quecksilber und/oder Eisen, gewünschtenfalls in Form eines Salzes, aktiviert ist, in einer Mischung von Wasser, Mineralsäure und einer aliphatischen $C_{1-3}$-Carbonsäure reduziert und danach - falls gewünscht - das erhaltene Produkt aus einem sauren Medium, vorzugsweise im pH-Bereich von 2 - 3, in kristalliner Form isoliert.

2. Verfahren nach Anspruch 1, dadurch gekennzeichnet, daß man als Mineralsäure Halogenwasserstoffsäure, vorzugsweise Bromwasserstoffsäure, und als aliphatische Carbonsäure Ameisensäure verwendet.

3. Verfahren nach Anspruch 1 oder 2, dadurch gekennzeichnet, daß man das aktivierend wirkende Metalladditiv wiedergewinnt und erneut verwendet.

4. 2-[(Methoxycarbonyl)-amino]-1H-benzimidazol-5(6)-thiol mit einem Schmelzpunkt von nahezu 435° C sowie dessen Salze, erhältlich durch Reduktion einer Verbindung der allgemeinen Formel II oder deren Salzen, worin A, X und $A^1$ die in Anspruch 1 angegebene Bedeutung haben und R = $CH_3$ ist, in der in Ansprüchen 1 bis 3 angegebenen Weise.

## Claims

1. Method for preparing benzimidazole-thiol-derivatives of general formula

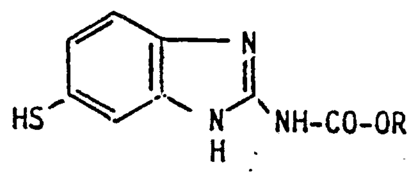

(I)

wherein

R represents $C_{1-5}$ alkyl,

by reducing a compound of general Formula II or salts thereof

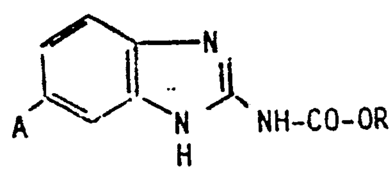

(II)

wherein

A represents $-SO_2X$, -SOH, $-SOA^1$ or $-SA^1$,

X represents chloride or hydrogen and

$A^1$ represents a group of general formula

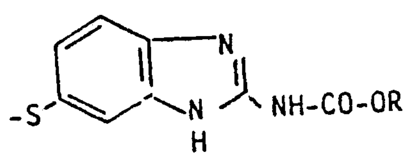

($A^1$)

and

R is defined as above,

by means of a metal in the presence of water and a mineral acid at a temperature ranging from 0 - 100 degrees C, characterised in that a compound of general Formula II, wherein A, X, $A^1$ and R are defined as above, is reduced, by means of aluminium which is activated up to 5 wt.%, based on aluminium, by tin, lead, magnesium, zinc, mercury and/or iron, optionally in the form of a salt, in a mixture of water, mineral acid and an aliphatic $C_{1-3}$ carboxylic acid and then, optionally, the product obtained is isolated, from an acidic medium, preferably in the pH range of 2-3, in crystalline form.

2. Method according to claim 1, characterised in that hydrohalic acid, preferably hydrobromic acid, is used as mineral acid and formic acid is used as aliphatic carboxylic acid.

3. Method according to either claim 1 or 2, characterised in that the metal additive which has the effect of an activator is recovered and reused.

4. 2-[(Methoxycarbonyl)-amino]-1H-benzimidazole-5(6)-thiol having a melting point of almost 435° C and the salts thereof, obtainable by reducing a compound of general Formula II or the salts thereof, wherein A, X and $A^1$ are defined as above and $R = CH_3$, using the method indicated in claims 1 to 3.

**Revendications**

1. Procédé de préparation de dérivés benzimidazole-thiol de la formule générale :

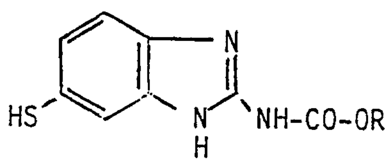

(I) ,

où

R représente un $C_{1-5}$ alkyle,
par la réduction d'un composé de la formule générale II

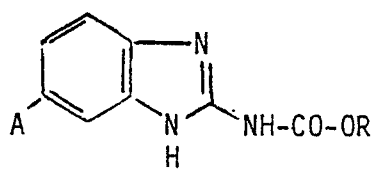

(II) ,

ou d'un sel de celui-ci
où

A est $-SO_2X$, -SOH $-SOA^1$ ou $-SA^1$,
X est le chlore ou l'hydrogène, et
$A^1$ est un groupe de la formule générale :

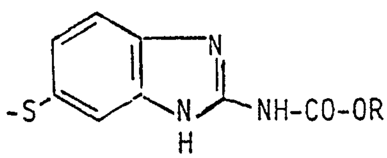

$(A^1)$

où R a la signification indiquée ci-dessus,
au moyen d'un métal en présence d'eau et d'un acide minéral à une température comprise entre 0 et 100°, caractérisé en ce que
on réduit un composé de la formule générale II,
où A, X, $A^1$ et R ont la signification donnée ci-dessus, à l'aide d'aluminium, lequel est activé avec jusqu'à 5%, ce pourcentage étant exprimé en poids par rapport à l'aluminium, d'étain, de plomb, de magnésium, de zinc, de mercure et/ou de fer, le cas échéant sous la forme d'un sel, en mélange avec de l'eau, un acide minéral et un acide carboxylique aliphatique en $C_{1-3}$ et ensuite, lorsque cela est souhaité, on isole le produit obtenu par cristallisation dans un milieu acide, ayant de préférence un pH compris entre 2 et 3.

2. Procédé selon la revendication 1, caractérisé en ce que l'on utilise en guise d'acide minéral un haloacide, de préférence l'acide bromhydrique, et en guise d'acide carboxylique aliphatique, l'acide formique.

3. Procédé selon la revendication 1 ou 2, caractérisé en ce que l'additif métallique agissant en tant qu'activateur est récupéré et réutilisé.

**4.** 2-[(méthoxycarbonyl)-amino]-1H-benzimidazole-5(6)-thiol avec un point de fusion approximativement de 435 °C, ainsi que ses sels, obtenus par la réduction d'un composé de la formule générale II ou d'un sel de celui-ci, où A, X et $A^1$ ont la signification donnée dans la revendication 1 et R = $CH_3$, conformément au procédé des revendications 1 à 3.